(19) **Europäisches Patentamt**
**European Patent Office**
**Office européen des brevets**

(11) **EP 4 070 714 B1**

(12) # EUROPEAN PATENT SPECIFICATION

(45) Date of publication and mention
of the grant of the patent:
**13.11.2024 Bulletin 2024/46**

(21) Application number: **22153533.9**

(22) Date of filing: **26.01.2022**

(51) International Patent Classification (IPC):
*A61B 1/00* *(2006.01)* *G06T 7/00* *(2017.01)*
*G06T 7/73* *(2017.01)* *G06V 20/50* *(2022.01)*
*G06V 10/25* *(2022.01)* *G06V 10/80* *(2022.01)*
*G06V 10/82* *(2022.01)*

(52) Cooperative Patent Classification (CPC):
**G06T 7/0012; A61B 1/000096; G06T 7/73;**
**G06V 10/25; G06V 10/806; G06V 10/811;**
**G06V 10/82; G06V 20/50;** G06T 2207/10016;
G06T 2207/10068; G06T 2207/20016;
G06T 2207/20076; G06T 2207/20081;
G06T 2207/20084; G06V 2201/031

(54) **TRACHEAL INTUBATION POSITIONING METHOD AND DEVICE BASED ON DEEP LEARNING, AND STORAGE MEDIUM**

VERFAHREN UND VORRICHTUNG ZUR POSITIONIERUNG EINER TRACHEALINTUBATION BASIEREND AUF DEEP LEARNING SOWIE EIN SPEICHERMEDIUM

PROCÉDÉ ET DISPOSITIF DE POSITIONNEMENT D'INTUBATION TRACHÉALE BASÉS SUR DEEP LEARNING ET SUPPORT D'ENREGISTREMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.02.2021 CN 202110196669**

(43) Date of publication of application:
**12.10.2022 Bulletin 2022/41**

(73) Proprietor: **Shanghai 9th People's Hospital, Shanghai Jiaotong**
**University School Of Medicine**
**Shanghai 200011 (CN)**

(72) Inventors:
• **JIANG, Hong**
**Shanghai 200011 (CN)**
• **XIA, Ming**
**Shanghai 200011 (CN)**
• **CHANG, Min**
**Shanghai 200011 (CN)**
• **ZHANG, Rong Fu**
**Shanghai 200011 (CN)**
• **LI, Feng**
**Shanghai 200011 (CN)**
• **XU, Tian Yi**
**Shanghai 200011 (CN)**

(74) Representative: **2K Patentanwälte Blasberg Kewitz & Reichel**
**Partnerschaft mbB**
**Schumannstrasse 27**
**60325 Frankfurt am Main (DE)**

(56) References cited:
**US-A1- 2015 059 736**

• **JOSEPH REDMON ET AL: "YOLOv3: An Incremental Improvement", ARXIV, CORNELL UNIVERSITY LIBRARY, 201 OLIN LIBRARY CORNELL UNIVERSITY ITHACA, NY 14853, 9 April 2018 (2018-04-09), pages 1 - 6, XP080868709**
• **YANGHAO LI ET AL: "Scale-Aware Trident Networks for Object Detection", ARXIV.ORG, CORNELL UNIVERSITY LIBRARY, 201 OLIN LIBRARY CORNELL UNIVERSITY ITHACA, NY 14853, 7 January 2019 (2019-01-07), XP081465590**
• **ANONYMOUS: "Apply Tridentnet with YOLO . Issue #10 . TuSimple/simpledet . GitHub", 31 January 2019 (2019-01-31), github.com, pages 1 - 3, XP055937662, Retrieved from the Internet <URL:https://github.com/TuSimple/simpledet/issues/10> [retrieved on 20220701]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

• REDMON JOSEPH ET AL: "You Only Look Once: Unified, Real-Time Object Detection", 2016 IEEE CONFERENCE ON COMPUTER VISION AND PATTERN RECOGNITION (CVPR), IEEE, 27 June 2016 (2016-06-27), pages 779 - 788, XP033021255, DOI: 10.1109/CVPR.2016.91

**Description**

**Field**

The present disclosure relates to the technical field of computer aided medical treatment, and particularly relates to a multi-modal tracheal intubation positioning method and device based on deep learning.

**Background**

Endotracheal intubation is an important method for anesthetists to perform airway management for patients in the general anesthesia state, and plays an important role in the aspects of maintaining unobstructed airway, ventilation and oxygen supply, respiratory support, and keeping oxygenation, etc. Anesthetists will face many challenges in the process of airway intubation, such as difficulty in mask ventilation and difficulty in intubation. According to relevant literature reports, in patients suffering from general anesthesia, the incidence of mask ventilation difficulty is about 0.9% to 12.8%, and the incidence of intubation difficulty is about 0.5% to 10%. At the same time, the incidence of simultaneous presence of mask ventilation difficulty and intubation difficulty is about 0.01% to 0.07%. Difficult or failed airway intubation often leads to serious consequences, including permanent brain injury or even death. For this reason, awake intubation under bronchofiberscope guidance is often used clinically to assist anesthetists in airway intubation for patients to ensure patient safety to the greatest extent.

In recent years, artificial intelligence technology has been rapidly developed and also has been preliminarily explored in the fields of medicine and anesthesia. In terms of tracheal intubation, more intelligent and automated intubation equipment has been initially developed. In 2012, Hemmerling et al., in Canada invented a remotely controlled tracheal intubation device-Kepler intubation system (KIS), which is the first robotic system for tracheal intubation. This operating system verified and implemented the possibility of remotely controlling the operation of tracheal intubation for the first time. Biro et al., in University of Zurich in Switzerland have researched and developed a robotic endoscope-automated via laryngeal imaging for tracheal intubation (REALITI), which has real-time image recognition and remote automatic positioning functions. An operator manually controls the bending movement of the tip of the endoscope.

When the glottis opening is detected by the image recognition, a user can hold a special button to activate an automatic mode. In the automatic mode, the tip of the endoscope moves to the geometric center point of the glottis opening until the tip enters the trachea.

US2015/ 059736A1 discloses an intubation system based on an airway pattern indicating a trachea opening. The airway pattern is determined from analysis of airway data detected by a trachea identifying device disposed on a moveable guide stylet of the intubation system. A navigation element is generated based on the airway pattern. In one embodiment, the airway pattern is a gas exchange pattern indicating a trachea opening. In another embodiment, the trachea opening transition pattern is a topographic pattern indicating a trachea opening. The guide stylet is capable of moving in a plurality of degrees of freedom in the airway following the guidance from the navigation element.

Although airway intubation technology has made many research progresses, most of them are still based on a single endoscopic image imaging method. In the intubation process, the viewing angle of the endoscopic image is relatively small, and the image contrast, target distance, target size and the like will all change, which is not conducive for a doctor to quickly lock the target. In addition, sputum and airway secretions can also block the tracheal orifice or the esophageal orifice and other targets, resulting in interference. Therefore, there is an urgent need for a method capable of quickly locking the target. JOSEPH REDMON ET AL: "YOLOv3: An Incremental Improvement" and YANGHAO LI ET AL: "Scale-Aware Trident Networks for Object Detection" show neural network architectures for obhject detection. "Apply Tridentnet with YOLO . Issue #10 . TuSimple/ simpledet. GitHub", https://github.com/TuSimple/simpledet/issues/10 shows how to combine the above neural network architectures.

**Summary**

The technical problem to be solved by the present disclosure is to provide a multi-modal tracheal intubation positioning method and device based on deep learning, which can quickly detect the tracheal orifice and the esophageal orifice can be rapidly detected in real

The present invention is set out in the appended set of claims.

Beneficial Effects

Due to the adoption of the above technical solutions, compared with the prior art, the present disclosure has the following advantages and positive effects: image information of the endoscope and carbon dioxide concentration information are fused, so that the detection effect of the tracheal orifice and the esophageal orifice is improved. According to the present disclosure, the Darknet53 backbone network of the traditional YOLOv3 is improved, a weight-shared parallel multiple branch dilated convolution residual block is constructed, and the capability of extracting the image feature by the backbone network is enhanced. Then, on the basis of retaining the original output layer of the YOLOv3, another two feature images in different scales are generated by the feature pyramid network, and the feature maps are subjected to upsampling and tensor splicing, so that the detection effect on small-size targets is improved. Meanwhile, the target center position is determined by a vectorized positioning algorithm based on the differences of four paths of carbon dioxide concentrations. Finally, the acquired target information and the target information acquired by the image are fused to determine the position of the trachea. Experiments have proved that compared with other methods, the present disclosure has the advantages that the detection accuracy for the tracheal orifice and the esophageal orifice is improved, and the multi-modal tracheal intubation auxiliary prototype device is feasible to perform tracheal intubation auxiliary guidance on a simulator, and has relatively satisfactory operation time and success rate.

**Brief Description of the Drawings**

FIG. 1 is a diagram of a hardware structure of a computer device for a tracheal intubation positioning method according to an embodiment of the present disclosure;
FIG. 2 is a flowchart of a first embodiment of the present disclosure;
FIG. 3 is a schematic diagram of a YOLOv3 network based on dilated convolution and feature fusion in the first embodiment of the present disclosure;
FIG. 4 is a schematic diagram of a residual module in the first embodiment of the present disclosure; and
FIG. 5 is a structural schematic diagram of a second embodiment of the present disclosure.

**Detailed Description of the Embodiments**

The present disclosure will be described in detail below in combination with specific embodiments. It should be understood that these embodiments are only used to describe the present disclosure and are not intended to limit the scope of the present disclosure.

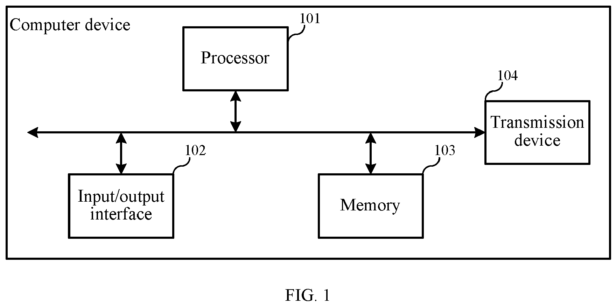

The embodiments of the present disclosure may be performed in a mobile device, a computer device, or a similar operation device (such as ECU) and system. By taking the computer device as an example, FIG. 1 is a diagram of a hardware structure of a computer device for a tracheal intubation positioning method. As shown in FIG. 1, the computer device may include one or more (only one shown in the figure) processors 101 (including but not limited to a central processing unit (CPU), a graphic processing unit (GPU), a digital signal processor (DSP), a microprogrammed control unit (MCU) or a field programmable gate array (FPGA), and other processing devices), an input/output interface 102 for interacting with a user, a memory 103 for storing data, and a transmission device 104 for a communication function. Those of ordinary skill in the art may understand that the structure shown in FIG. 1 is only schematic and does not limit the structure of the above electronic device. For example, the computer device may further include more or fewer components than those shown in FIG. 1, or have a different configuration from that shown in FIG. 1.

The input/output interface 102 may be connected to one or more displays, touch screens and the like to display data transmitted from the computer device, and may also be connected to a keyboard, a stylus, a touchpad and/or a mouse, etc., to input user instructions such as selection, creation, or edition.

The memory 103 may be configured to store a software program for storing application software and a module, for example, a program instruction/module corresponding to a tracheal intubation positioning method in an embodiment of the present disclosure. The processor 101 runs the software program and module stored in the memory 103 so as to perform various functional applications and data processing, that is, the above tracheal intubation positioning method is implemented. The memory 103 may include a high-speed random access memory, and may further include a non-volatile memory, such as one or more magnetic storage devices, flash memories, or other non-volatile solid-state memories. In some instances, the memory 103 may further include memories which are arranged remotely relative to the processor 101. These remote memories may be connected to the computer device through networks. The instances of the above networks include, but are not limited to, Internet, Intranet, a local area network, a mobile communication network and a combination thereof.

The transmission device 104 is configured to receive or transmit data through a network. The specific instance of the above network may include the Internet provided by a communication provider of the computer device. Under the above running environment, the present disclosure provides a tracheal intubation positioning method.

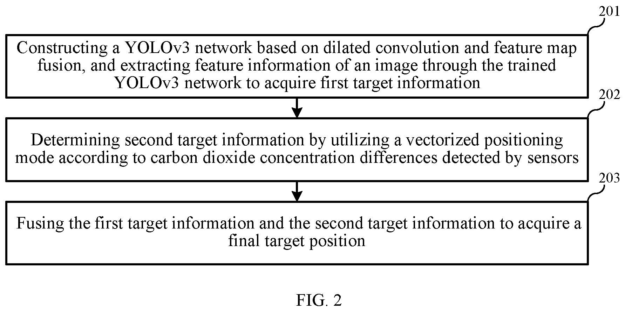

FIG. 2 shows a flowchart of a tracheal intubation positioning method according to an embodiment of the present disclosure. The method specifically includes the following steps.

Step 201: a YOLOv3 network based on dilated convolution and feature map fusion is constructed, and feature information of an endoscopic image is extracted through the YOLOv3 network to acquire first target information.

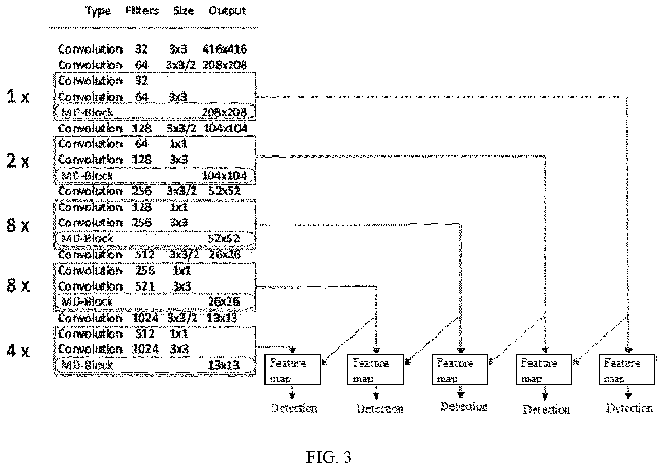

Specifically, in the tracheal intubation process, a target scale changes greatly, and medium-scale and small-scale target semantic information in a deep network may be lost. However, the size of a convolution kernel in the backbone network of the traditional YOLOv3 is fixed, and the capability of extracting image feature information is limited. Therefore, the embodiment provides the YOLOv3 network based on dilated convolution and feature fusion, as shown in FIG. 3.

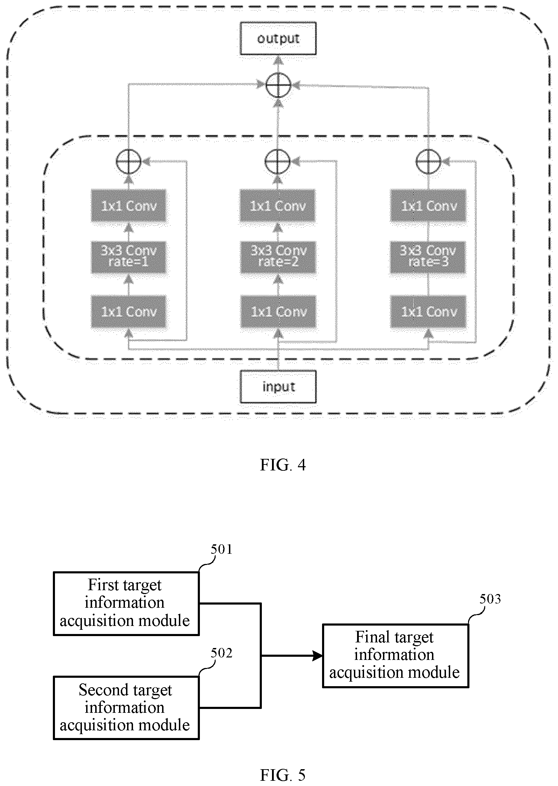

Firstly, the backbone network Darknet53 of the YOLOv3 is improved, and weight-shared parallel multiple branch dilated convolution blocks (MD-Blocks) are designed to extract richer features of an image, as shown in FIG. 4. The block uses dilated convolution kernels with different expansion rates to extract target feature information in different scales; meanwhile, the number of the feature maps is increased by virtue of upsampling and tensor splicing technologies, and the precision of detecting the small target is improved. The original residual block is replaced with three parallel residual blocks, and 1x1 convolution kernels are added to the head and tail of each residual block so that the invariant channel number is ensured. Meanwhile, the 3x3 original ordinary convolution is replaced with three 3x3 dilated convolutions with different expansion rates, and the weights of the dilated convolutions in the three parallel residual blocks are shared. In the embodiment, the residual blocks in the backbone network Darnet53 are all replaced with the designed weight-shared parallel MD-Blocks.

Secondly, in order to further detect shallower features, another two feature maps in different scales are generated by a feature pyramid network on the basis of maintaining the original output layer of the YOLOv3. The specific process is as follows: an output feature map with a size of 52x52 is subjected to upsampling and is subjected to tensor splicing with the output of a shallow convolution layer of 104x104, and thus a feature map with a size of 104x104 is output. Similarly, the output feature map with the size of 104x104 is subjected to upsampling and is subjected to tensor splicing with the output of a convolution layer 208x208 in the backbone network, and thus a feature map with a size of 208x208 is output. Table 1 lists the parameter configuration of the weight-shared parallel MD-Blocks.

Table 1 Parameter configuration of the weight-shared parallel MD-Blocks

| Block output, the number of the channels is n | | |
|---|---|---|
| 1x1 convolution, the number of the channels is n | 1x1 convolution, the number of the channels is n | 1x1 convolution, the number of the channels is n |
| 3x3 dilated convolution, the expansion rate is 1 | 3x3 dilated convolution, the expansion rate is 2 | 3x3 dilated convolution, the expansion rate is 3 |
| The number of the channels is n/4 | The number of the channels is n/4 | The number of the channels is n/4 |
| 1x1 convolution, the number of the channels is n/4 | 1x1 convolution, the number of the channels is n/4 | 1x1 convolution, the of the channels is n/4 |
| Block input, the number of the channels is n | | |

In the embodiment, a mean square error is adopted for the center coordinate, width, and height of a bounding box predicted by the YOLOv3 network. Meanwhile, during classification, a Softmax classification function is replaced with a plurality of logistic regressions, and the classification loss and the confidence loss of the bounding box are calculated by a binary cross-entropy function. Assuming that the size of the acquired feature map is SxS, each grid generates B anchor boxes, SxSxB bounding boxes are obtained by each preselection box via the network, and a final loss function $L_{total}$ includes a detection box center coordinate error loss $L_{mid}$, a detection box height and width error loss $L_{margin}$, a confidence error loss $L_{conf}$ and a classification error loss $L_{class}$. It is defined that if the intersection over the union of a certain preselection box to a ground true box is greater than the intersection over the union of other preselection boxes to the ground true box, a current target is detected by adopting the certain preselection box.

$$L_{mid} = \lambda_{coord} \sum_{i=0}^{s^2} \sum_{j=0}^{B} I_{ij}^{obj} \left[ \left( x_i^j - \hat{x}_i^j \right)^2 + \left( y_i^j - \hat{y}_i^j \right)^2 \right]$$

$$L_{m\arg in} = \lambda_{coord} \sum_{i=0}^{s^2} \sum_{j=0}^{B} I_{ij}^{obj} \left[ \left( \sqrt{w_i^j} - \sqrt{\hat{w}_i^j} \right)^2 + \left( \sqrt{h_i^j} - \sqrt{\hat{h}_i^j} \right)^2 \right]$$

$$L_{conf} = -\sum_{i=0}^{s^2} \sum_{j=0}^{B} I_{ij}^{obj} \left[ \hat{c}_i^j \log\left( c_i^j \right) + \left( 1 - \hat{c}_i^j \right) \log\left( 1 - c_i^j \right) \right]$$

$$- \lambda_{noobj} \sum_{i=0}^{s^2} \sum_{j=0}^{B} I_{ij}^{noobj} \left[ \hat{c}_i^j \log\left( c_i^j \right) + \left( 1 - \hat{c}_i^j \right) \log\left( 1 - c_i^j \right) \right]$$

$$L_{class} = -\sum_{i=0}^{s^2} I_{ij}^{obj} \sum_{c \in O} \left( \left[ \hat{p}_i^j \log\left( p_i^j \right) + \left( 1 - \hat{p}_i^j \right) \log\left( 1 - p_i^j \right) \right] \right)$$

$$L_{total} = L_{mid} + L_{m\arg in} + L_{conf} + L_{class}$$

In the above formulas, $x_i^j, y_i^j, w_i^j, h_i^j$ respectively represent the center coordinate, width, and height of the bounding box output by the network; $\hat{x}_i^j, \hat{y}_i^j, \hat{w}_i^j, \hat{h}_i^j$ respectively represent the center coordinate, width and height of the true box; $\lambda_{coord}$, $\lambda_{noobj}$ are various hyperparameters; and $I_{ij}^{obj}$ represents whether a $j$th preselection box of an $i$th network is responsible for detecting the current target, with a value of 1 or 0. $I_{ij}^{noobj}$ represents that the $j$th preselection box of the $i$th network is not responsible for detecting the current target; $\hat{c}_i^j$ represents that the confidence of the target truly exists in the $j$th preselection box of the $i$th network; $c_i^j$ represents that the confidence of the target exists in the $j$th preselection box of the $i$th network through detection; $O$ represents a set of all to-be-detected categories; $c$ represents the currently detected category; $\hat{p}_i^j$ represents a probability that the category which is an object truly exists in the $j$th preselection box of the $i$th network; and $p_i^j$ represents a probability that the category which is an object exists in the $j$th preselection box of the $i$th network through detection.

In the process of training the improved network, training parameters are correspondingly configured in the embodiment. Specifically, a size of batch is set to 4, subdivisions are set to 8, acquired 80 images are equally divided into 8 groups to be trained respectively, weight decay is set to 0.0005, and a momentum is set to 0.9. In the later stage of training, a learning decay strategy is set to step, a learning rate change factor is set to 0.1, and the parameters of the network are updated by a stochastic gradient descent (SGD) method.

Step 202: second target information is determined by utilizing a vectorized positioning mode according to carbon dioxide concentration differences detected by sensors.

Specifically, in the embodiment, the target center position is determined by a vectorized positioning algorithm based on the differences of four paths of carbon dioxide concentrations. The specific method is as follows: a Cartesian coordinate system is established by calibrating the positions of the sensors for carbon dioxide according to the mounting positions of the sensors for the four paths of carbon dioxide. Carbon dioxide concentration vectors are measured by a sensor 1, a sensor 2, a sensor 3 and a sensor 4 are respectively $OC1$, $OC2$, $OC3$, $OC4$, and $\theta$ is an included angle between $OC1$ or $OC3$ and an $x$ axis in the Cartesian coordinate system or an included angle between $OC2$ or $OC4$ and a $y$ axis in the Cartesian coordinate system, the coordinate position (x0,y0) of the target center point is calculated according to the established coordinate system based on the following formula:

$$x_0 = \frac{(OC1 - OC3) * \cos\theta + (OC4 - OC2) * \sin\theta}{\delta}$$

$$y_0 = \frac{(OC1 - OC3) * \sin\theta + (OC4 - OC2) * \cos\theta}{\delta}$$

wherein $\delta$ is a normalization factor.

Step 203: the first target information and the second target information are fused to acquire a final target position. That is, a transformation relationship between an image coordinate system and a carbon dioxide vectorized positioning coordinate system (that is, the Cartesian coordinate system) is established, and the target center position (that is, the second target information) calculated by the vectorized positioning method based on the differences of the multiple paths of carbon dioxide concentrations is mapped to the image coordinate system to be marked as $(b_{cx}, b_{cy})$. Further, the $(b_{cx}, b_{cy})$ and the center coordinate (that is, the first target information) of the bounding box calculated by the improved YOLOv3 network model based on dilated convolution and feature fusion are subjected to weighted fusion to finally obtain the accurate target center coordinate.

Specifically, four offsets $t_x, t_y, t_w, t_h$ are predicted for each bounding box through the improved YOLOv3 network, which respectively represents a center coordinate of a predicted target object, and a width and a height of a target preselection box. In addition, the network also outputs a probability value of measuring the presence of the target object in the preselection box, and the category of the target object. Assuming that the grid where the target object is located offsets from the upper left corner of the image, the offset length and width are respectively $c_x, c_y$, and the width and height of the preselection box are respectively $p_w, p_h$. The center coordinate information of the target bounding box predicted by the network under the image coordinate system is obtained by the following computational formula:

$$b_{ix} = \sigma(t_x) + c_x$$

$$b_{iy} = \sigma(t_y) + c_y$$

wherein $\sigma(\ )$ represents a sigmoid function.

Further, the weighted fusion is performed on the target center coordinate (that is, the first target information) of the target bounding box predicted by the network and the coordinate $(b_{cx}, b_{cy})$ obtained by mapping the target center position (that is, the second target information) calculated by the vectorized positioning algorithm based on the differences of the multiple paths of carbon dioxide concentrations to the image coordinate system to obtain the center coordinate of the final target box:

$$b_x = \alpha b_{ix} + \beta b_{cx}$$

$$b_y = \alpha b_{iy} + \beta b_{cy}$$

$$b_w = p_w e^{t_w}$$

$$b_h = p_h e^{t_h}$$

wherein $b_x, b_y, b_w, b_h$ respectively represent the center coordinate, width, and height of the finally calculated target bounding box, and $\alpha, \beta$ respectively present weight factors.

FIG. 5 shows a structural schematic diagram of a tracheal intubation positioning device according to a second embodiment of the present disclosure. The device is configured to perform the method process as shown in FIG. 2, and the device includes a first target information acquisition module 501, a second target information acquisition module 502, and a final target position acquisition module 503.

The first target information acquisition module 501 is configured to construct a YOLOv3 network based on dilated convolution and feature map fusion, and extract feature information of an endoscopic image through the trained YOLOv3 network to acquire first target information, wherein the constructed YOLOv3 network adopts a residual module to extract target feature information in different scales of the endoscopic image; the residual module includes three parallel residual blocks, and 1x1 convolution kernels are added to the head and tail of each residual block; and the three parallel residual blocks have different expansion rates, and the weights of the dilated convolutions in the three parallel residual blocks are shared. An output layer of the YOLOv3 network generates two feature maps in different scales through a feature pyramid network. Generating the feature maps through the feature pyramid network refers to upsampling the feature map output by this convolution layer and performing tensor splicing with the output of the last convolution layer in the network to acquire a feature map. A loss function of the YOLOv3 network includes a detection box center coordinate error loss, a detection box height and width error loss, a confidence error loss, and a classification error loss. The second target information acquisition module 502 is configured to determine second target information by utilizing a vectorized positioning mode according to carbon dioxide concentration differences detected by sensors. The final target position acquisition module 503 is configured to fuse the first target information and the second target information to acquire a final target position.

16 resident doctors in grades 1 to 2 who were in standardized training in the Department of Anesthesiology, Shanghai Ninth People's Hospital of Shanghai Jiao Tong University School of Medicine in October 2020, were selected as experimental subjects. These 16 resident doctors had experience in nasal/orotracheal intubation, but had no experience in using the embodiments of the present disclosure. All the 16 resident doctors completed 40 operation exercises on a simulator having a difficult airway, and all operation records were completely recorded. Among the 640 operations performed by all the resident doctors, the average operation time is $30.39 \pm 29.39s$, the longest time is 310s, the number of successful operations is 595, and the success rate is 93%.

It is not difficult to find that image information of the endoscope and carbon dioxide concentration information are fused, so that the detection effect of the tracheal orifice and the esophageal orifice is improved. According to the present disclosure, the Darknet53 backbone network of the traditional YOLOv3 is improved, a weight-shared parallel multiple branch dilated convolution residual module is constructed, and the capability of extracting the image feature by the backbone network is enhanced. Then, on the basis of retaining the original output layer of the YOLOv3, another two feature images in different scales are generated by the feature pyramid network, and the feature maps are subjected to upsampling and tensor splicing, so that the detection effect on small-size targets is improved. Meanwhile, the target center position is determined by a vectorized positioning algorithm based on the differences of four paths of carbon dioxide concentrations. Finally, the acquired target information and the target information acquired by the image are fused to determine the position of the trachea. Experiments prove that compared with other methods, the present disclosure has the advantages that the detection accuracy for the tracheal orifice and the esophageal orifice is improved, and a multi-modal tracheal intubation auxiliary prototype device is feasible to perform tracheal intubation auxiliary guidance on a simulator, and has relatively satisfactory operation time and success rate.

**Claims**

1. A multi-modal tracheal intubation positioning method based on deep learning, comprising the following steps:

   (1) constructing a YOLOv3 network based on dilated convolution and feature map fusion, and extracting feature information of an endoscopic image through the trained YOLOv3 network to acquire first target information;
   (2) determining second target information by utilizing a vectorized positioning mode according to carbon dioxide concentration differences detected by sensors; wherein there are totally four sensors in the step (2); and the step of establishing a Cartesian coordinate system by calibrating the position of each sensor and determining the second target information according to the coordinate system is specifically as follows:

$$x_0 = \frac{(OC1 - OC3) * \cos\theta + (OC4 - OC2) * \sin\theta}{\delta}$$

$$y_0 = \frac{(OC1 - OC3) * \sin\theta + (OC4 - OC2) * \cos\theta}{\delta},$$

   wherein $OC1$, $OC2$, $OC3$, $OC4$ are respectively carbon dioxide concentration vectors measured by the four sensors, $\theta$ is an included angle between $OC1$ or $OC3$ and an $x$ axis in the Cartesian coordinate system or an included angle between $OC2$ or $OC4$ and a $y$ axis in the Cartesian coordinate system, and $\delta$ is a normalization

factor;and

(3) fusing the first target information and the second target information to acquire a final target position, wherein the step (3) is specifically as follows: performing weighted fusion on a center coordinate of a bounding box of the first target information and a center position obtained by mapping the center position of the second target information to an image coordinate system to acquire the final target position.

2. The multi-modal tracheal intubation positioning method based on deep learning according to claim 1, wherein the YOLOv3 network in the step (1) adopts a residual module to extract target feature information in different scales of the endoscopic image; the residual module comprises three parallel residual blocks, and 1x1 convolution kernels are added to the head and tail of each residual block; and the three residual blocks have different expansion rates, and the weights of the dilated convolutions in the three parallel residual blocks are shared.

3. The multi-modal tracheal intubation positioning method based on deep learning according to claim 1, wherein an output layer of the YOLOv3 network in the step (1) generates two feature maps in different scales through a feature pyramid network.

4. The multi-modal tracheal intubation positioning method based on deep learning according to claim 3, wherein generating the feature maps through the feature pyramid network refers to upsampling the feature map output by this convolution layer and performing tensor splicing with the output of the last convolution layer in the network to acquire a feature map.

5. The multi-modal tracheal intubation positioning method based on deep learning according to claim 1, wherein a loss function of the YOLOv3 network in the step (1) comprises a detection box center coordinate error loss, a detection box height, and width error loss, a confidence error loss and a classification error loss.

6. A multi-modal tracheal intubation positioning device based on deep learning, comprising: a first target information acquisition module, configured to construct a YOLOv3 network based on dilated convolution and feature map fusion, and extract feature information of an image through the trained YOLOv3 network to acquire first target information; a second target information acquisition module, configured to determine second target information by utilizing a vectorized positioning mode according to carbon dioxide concentration differences detected by four sensors, establishing a Cartesian coordinate system by calibrating the position of each sensor and determining the second target information according to the coordinate system is specifically as

$$x_0 = \frac{(OC1 - OC3) * \cos\theta + (OC4 - OC2) * \sin\theta}{\delta}$$

follows: $y_0 = \frac{(OC1 - OC3) * \sin\theta + (OC4 - OC2) * \cos\theta}{\delta}$ , wherein $OC1$, $OC2$, $OC3$, $OC4$ are respectively carbon dioxide concentration vectors measured by the four sensors, $\theta$ is an included angle between $OC1$ or $OC3$ and an $x$ axis in the Cartesian coordinate system or an included angle between $OC2$ or $OC4$ and a $y$ axis in the Cartesian coordinate system, and $\delta$ is a normalization factor; and a final target position acquisition module, configured to fuse the first target information and the second target information to acquire a final target position, performing weighted fusion on a center coordinate of a bounding box of the first target information and a center position obtained by mapping the center position of the second target information to an image coordinate system to acquire the final target position.

7. A computer readable storage medium, wherein the computer readable storage medium stores a computer program; and when the computer program is executed by a processor, the multi-modal tracheal intubation positioning method according to any one of claims 1 to 5 is implemented.

**Patentansprüche**

1. Ein Multimodales, auf Deep Learning basierendes Verfahren zur Positionierung der trachealen Intubation, das die folgenden Schritte umfasst:

(1) Aufbau eines YOLOv3-Netzes auf der Grundlage von erweiterter Faltung und Merkmalskartenfusion und Extraktion von Merkmalsinformationen eines endoskopischen Bildes durch das trainierte YOLOv3-Netz, um erste Zielinformationen zu erfassen;

(2) Bestimmen einer zweiten Zielinformation unter Verwendung eines vektorisierten Positionierungsmodus gemäß den von den Sensoren erfassten Kohlendioxid-Konzentrationsdifferenzen; wobei es in dem Schritt (2) insgesamt vier Sensoren gibt; und der Schritt des Einrichtens eines kartesischen Koordinatensystems durch Kalibrieren der Position jedes Sensors und des Bestimmens der zweiten Zielinformation gemäß dem Koordinatensystem

$$x_0 = \frac{(OC1 - OC3) * \cos\theta + (OC4 - OC2) * \sin\theta}{\delta}$$

$$y_0 = \frac{(OC1 - OC3) * \sin\theta + (OC4 - OC2) * \cos\theta}{\delta}$$

insbesondere wie folgt ist: wobei $OC1$, $OC2$, $OC3$, $OC4$ jeweils von den vier Sensoren gemessene Kohlendioxid-Konzentrationsvektoren sind, $\theta$ ein eingeschlossener Winkel zwischen $OC1$ oder $OC3$ und einer $x$-Achse in dem kartesischen Koordinatensystem oder ein eingeschlossener Winkel zwischen $OC2$ oder $OC4$ und einer $y$-Achse in dem kartesischen Koordinatensystem ist, und $\delta$ ein Normalisierungsfaktor ist; und

(3) Verschmelzen der ersten Zielinformation und der zweiten Zielinformation, um eine endgültige Zielposition zu erhalten, wobei der Schritt (3) insbesondere wie folgt ist: Durchführen einer gewichteten Verschmelzung an einer Mittelkoordinate eines Begrenzungsrahmens der ersten Zielinformation und einer Mittelposition, die durch Abbilden der Mittelposition der zweiten Zielinformation auf ein Bildkoordinatensystem erhalten wird, um die endgültige Zielposition zu erhalten.

2. Das Multimodale, auf Deep Learning basierende Verfahren zur Positionierung der trachealen Intubation nach Anspruch 1, wobei das YOLOv3-Netzwerk im Schritt (1) ein Restmodul verwendet, um Zielmerkmalinformationen in verschiedenen Maßstäben des endoskopischen Bildes zu extrahieren; das Restmodul drei parallele Restblöcke umfasst und 1x1-Faltungskerne zum Kopf und Schwanz jedes Restblocks hinzugefügt werden; und die drei Restblöcke unterschiedliche Expansionsraten haben und die Gewichte der dilatierten Faltungen in den drei parallelen Restblöcken gemeinsam genutzt werden.

3. Das Multimodale, auf Deep Learning basierende Verfahren zur Positionierung der trachealen Intubation nach Anspruch 1 , wobei eine Ausgabeschicht des YOLOv3-Netzes im Schritt (1) zwei Merkmalskarten in unterschiedlichen Maßstäben durch ein Merkmalspyramidennetz erzeugt.

4. Das Multimodale, auf Deep Learning basierende Verfahren zur Positionierung der trachealen Intubation nach Anspruch 3, wobei sich das Erzeugen der Merkmalskarten durch das Merkmalspyramidennetzwerk auf das Upsampling der von dieser Faltungsschicht ausgegebenen Merkmalskarte und das Durchführen von Tensorsplicing mit der Ausgabe der letzten Faltungsschicht im Netzwerk bezieht, um eine Merkmalskarte zu erhalten.

5. Das Multimodale, auf Deep Learning basierendes Verfahren zur Positionierung der trachealen Intubation nach Anspruch 1, wobei eine Verlustfunktion des YOLOv3-Netzwerks im Schritt (1) einen Erkennungsbox-Mittelkoordinaten-Fehlerverlust, einen Erkennungsbox-Höhen-und -Breiten-Fehlerverlust, einen Konfidenzfehlerverlust und einen Klassifikationsfehlerverlust umfasst.

6. Multimodale Tracheal-Intubations-Positionierungsvorrichtung auf der Grundlage von Deep Learning, umfassend: ein erstes Zielinformationserfassungsmodul, das so konfiguriert ist, dass es ein YOLOv3-Netzwerk auf der Grundlage von dilatierter Faltung und Merkmalskartenfusion konstruiert und Merkmalsinformationen eines Bildes durch das trainierte YOLOv3-Netzwerk extrahiert, um erste Zielinformationen zu erfassen; ein zweites Zielinformationserfassungsmodul, das so konfiguriert ist, dass es zweite Zielinformationen durch Verwendung eines vektorisierten Positionierungsmodus gemäß Kohlendioxid-Konzentrationsunterschieden, die von vier Sensoren erfasst werden, bestimmt,ein kartesisches Koordinatensystem durch Kalibrieren der Position jedes Sensors einrichtet und die zweiten Zielinformationen gemäß dem Koordinatensystem

$$x_0 = \frac{(OC1 - OC3) * \cos\theta + (OC4 - OC2) * \sin\theta}{\delta}$$

$$y_0 = \frac{(OC1 - OC3) * \sin\theta + (OC4 - OC2) * \cos\theta}{\delta}$$

bestimmt, und zwar wie folgt: wobei $OC1$, $OC2$, $OC3$, $OC4$ jeweils von den vier Sensoren gemessene Kohlendioxid-Konzentrationsvektoren sind, $\theta$ ein eingeschlossener Winkel zwischen $OC1$ oder $OC3$ und einer $x$-Achse in dem kartesischen Koordinatensystem oder ein eingeschlossener Winkel zwischen $OC2$ oder $OC4$ und einer $y$-Achse in dem kartesischen Koordinatensystem ist, und $\delta$ ein Normalisierungsfaktor ist; und ein Modul zur Erfassung der endgültigen Zielposition, das so konfiguriert ist, dass es die ersten Zielinformationen und die zweiten Zielinformationen fusioniert, um eine endgültige Zielposition zu erfassen, wobei es eine gewichtete Fusion an einer Mittelkoordinate eines Begrenzungsrahmens der ersten Zielinformationen und einer Mittelposition durchführt, die durch Abbilden der Mittelposition der zweiten Zielinformationen auf ein Bildkoordinatensystem erhalten wird, um die endgültige Zielposition zu erfassen.

**7.** Computerlesbares Speichermedium, wobei das computerlesbare Speichermedium ein Computerprogramm speichert; und wenn das Computerprogramm von einem Prozessor ausgeführt wird, wird das multimodale Trachealintubations-Positionierungsverfahren nach einem der Ansprüche 1 bis 5 implementiert.

**Revendications**

**1.** Un procédé multimodal basé sur l'apprentissage profond pour le positionnement de l'intubation trachéale, qui comprend les étapes suivantes :

(1) Construction d'un réseau YOLOv3 basé sur le repliement étendu et la fusion de cartes de caractéristiques et extraction d'informations de caractéristiques d'une image endoscopique par le réseau YOLOv3 entraîné afin de capturer les premières informations cibles ;
(2) déterminer une seconde information cible en utilisant un mode de positionnement vectorisé selon les différences de concentration de dioxyde de carbone détectées par les capteurs ; dans lequel il y a quatre capteurs au total dans l'étape (2) ; et l'étape d'établissement d'un système de coordonnées cartésiennes en étalonnant la position de chaque capteur et en déterminant la seconde information cible selon le système de coordonnées est en particulier comme suit :

$$x_0 = \frac{(OC1 - OC3) * \cos\theta + (OC4 - OC2) * \sin\theta}{\delta}$$

$$y_0 = \frac{(OC1 - OC3) * \sin\theta + (OC4 - OC2) * \cos\theta}{\delta}$$

où $OC1$, $OC2$, $OC3$, $OC4$ sont des vecteurs de concentration de dioxyde de carbone mesurés respectivement par les quatre capteurs, $\theta$ est un angle inclus entre $OC1$ ou $OC3$ et un axe $x$ dans le système de coordonnées cartésiennes ou un angle inclus entre $OC2$ ou $OC4$ et un axe $y$ dans le système de coordonnées cartésiennes, et $\delta$ est un facteur de normalisation ; et
(3) fusion des premières informations cibles et des secondes informations cibles pour obtenir une position cible finale, l'étape (3) étant en particulier la suivante : réalisation d'une fusion pond érée à une coordonnée centrale d'un cadre de délimitation des premières informations cibles et une position centrale obtenue en mappant la position centrale des secondes informations cibles sur un système de coordonnées d'image pour obtenir la position cible finale.

**2.** Le procédé multimodal basé sur l'apprentissage profond pour le positionnement de l'intubation trachéale selon la revendication 1, dans lequel le réseau YOLOv3 utilise à l'étape (1) un module de résidus pour extraire des informations de caractéristiques cibles à différentes échelles de l'image endoscopique ; le module résiduel comprend trois blocs résiduels parallèles et des noyaux de plis 1x1 sont ajoutés à la tête et à la queue de chaque bloc résiduel ; et les trois blocs résiduels ont des taux d'expansion différents et les poids des plis dilatés dans les trois blocs résiduels parallèles sont partagés.

3. Le procédé multimodal basé sur l'apprentissage profond pour le positionnement de l'intubation trachéale selon la revendication 1 , dans lequel une couche de sortie du réseau YOLOv3 génère à l'étape (1) deux cartes de caractéristiques à différentes échelles par un réseau pyramidal de caractéristiques.

4. Le procédé multimodal basé sur l'apprentissage profond pour le positionnement de l'intubation trachéale selon la revendication 3, dans lequel la génération des cartes de caractéristiques par le réseau de pyramides de caractéristiques se rapporte au suréchantillonnage de la carte de caractéristiques émise par cette couche de convolution et à l'exécution d'un torso splicing avec la sortie de la dernière couche de convolution dans le réseau pour obtenir une carte de caractéristiques.

5. Le procédé multimodal basé sur l'apprentissage profond pour le positionnement de l'intubation trachéale selon la revendication 1, dans lequel une fonction de perte du réseau YOLOv3 à l'étape (1) comprend une perte d'erreur de coordonnées centrales de la boite de détection, une perte d'erreur de hauteur et de largeur de la boite de détection, une perte d'erreur de confiance et une perte d'erreur de classification.

6. Un dispositif de positionnement d'intubation trachéale multimodal basé sur l'apprentissage profond, comprenant : un premier module d'acquisition d'informations de cible configuré pour construire un réseau YOLOv3 sur la base d'une convolution dilatée et d'une fusion de cartes de caractéristiques, et pour extraire des informations de caractéristiques d'une image par le réseau YOLOv3 entraîné afin d'acquérir des premières informations de cible ; un deuxième module d'acquisition d'informations de cible configuré pour déterminer des deuxièmes informations de cible en utilisant un mode de positionnement vectorisé selon des différences de concentration de dioxyde de carbone détectées par quatre capteurs, établir un système de coordonnées cartésiennes en étalonnant la position de chaque capteur et déterminer les deuxièmes informations de cible

$$x_0 = \frac{(OC1 - OC3) * \cos\theta + (OC4 - OC2) * \sin\theta}{\delta}$$

$$y_0 = \frac{(OC1 - OC3) * \sin\theta + (OC4 - OC2) * \cos\theta}{\delta}$$

selon le système de coordonnées, comme suit : où $OC1$, $OC2$, $OC3$, $OC4$ sont respectivement des vecteurs de concentration de dioxyde de carbone mesurés par les quatre capteurs, $\theta$ est un angle inclus entre $OC1$ ou $OC3$ et un axe $x$ dans le système de coordonnées cartésiennes ou un angle inclus entre $OC2$ ou $OC4$ et un axe $y$ dans le système de coordonnées cartésiennes, et $\delta$ est un facteur de normalisation ; et un module de détection de position cible finale configuré pour fusionner les premières informations cibles et les deuxièmes informations cibles pour détecter une position cible finale, en effectuant une fusion pondérée à une coordonnée centrale d'un cadre de délimitation des premières informations cibles et une position centrale obtenue en mappant la position centrale des deuxièmes informations cibles sur un système de coordonnées d'image pour détecter la position cible finale.

7. Support de stockage lisible par ordinateur, dans lequel le support de stockage lisible par ordinateur stocke un programme informatique ; et lorsque le programme informatique est exécuté par un processeur, le procédé de positionnement multimodal d'intubation trachéale selon l'une quelconque des revendications 1 à 5 est mis en oeuvre.

FIG. 1

201

Constructing a YOLOv3 network based on dilated convolution and feature map fusion, and extracting feature information of an image through the trained YOLOv3 network to acquire first target information

202

Determining second target information by utilizing a vectorized positioning mode according to carbon dioxide concentration differences detected by sensors

203

Fusing the first target information and the second target information to acquire a final target position

FIG. 2

FIG. 3

1x1 Conv

3x3 Conv
rate=1

1x1 Conv

1x1 Conv

3x3 Conv
rate=2

1x1 Conv

1x1 Conv

3x3 Conv
rate=3

1x1 Conv

input

FIG. 4

501

First target
information
acquisition module

502

Second target
information
acquisition module

503

Final target
information
acquisition module

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015059736 A1

**Non-patent literature cited in the description**

- **JOSEPH REDMON et al.** *YOLOv3: An Incremental Improvement*
- **YANGHAO LI et al.** *Scale-Aware Trident Networks for Object Detection*
- *Apply Tridentnet with YOLO . Issue #10 . TuSimple/ simpledet. GitHub, https://github.com/TuSimple/sim-pledet/issues/10*